**Europäisches Patentamt**

**European Patent Office**

**Office européen des brevets**

(11) Veröffentlichungsnummer: **0 144 525**
**A2**

## (12) EUROPÄISCHE PATENTANMELDUNG

(21) Anmeldenummer: **84109542.5**

(22) Anmeldetag: **10.08.84**

(51) Int. Cl.⁴: **A 61 M 25/00**

(30) Priorität: **27.10.83 DE 3338909**

(43) Veröffentlichungstag der Anmeldung:
**19.06.85 Patentblatt 85/25**

(84) Benannte Vertragsstaaten:
**AT BE CH DE FR GB IT LI LU NL SE**

(71) Anmelder: **INTERMEDICAT GMBH**
**Gerliswilstrasse 74**
**CH-6020 Emmenbrücke(CH)**

(72) Erfinder: **Fuchs, Jürgen**
**Wolfhager Strasse 45**
**D-3501 Emstal(DE)**

(74) Vertreter: **von Kreisler, Alek, Dipl.-Chem. et al,**
**Deichmannhaus am Hauptbahnhof**
**D-5000 Köln 1(DE)**

(54) Katheteransatz.

(57) Bei dem erfindungsgemäßen Katheteransatz (1) ist vorgesehen, daß das Gehäuse (2) als einteiliges Kunststoffspritzgußteil um von Öffnungen (4,6) freie Bereiche eines mehrlumigen Katheters (3) und eines Anschlußschlauches (5) herumgeformt und mit einer nach außen abgedichteten Kammer (7) ausgestattet ist, in der das innere Ende des Anschlußschlauches (5) und eine Wandlochung (4) des mehrlumigen Katheters (3) angeordnet sind. Zwei gegenüberliegende Fenster (9) der Kammer (7) sind durch Deckel (10) dicht verschlossen. Diese Ausbildung des Katheteransatzes (1) ermöglicht seine einfache und preiswerte Herstellung im Spritzgußverfahren. Verklebungen von Öffnungen (4,6) des Katheters (3) und/oder des Anschlußschlauches (5) werden garantiert vermieden.

FIG.1

EP 0 144 525 A2

0144525

Katheteransatz

Die Erfindung bezieht sich auf einen Katheteransatz, bestehend aus einem Gehäuse, das einen mehrlumigen Katheter und mindestens einen zu diesem parallelen Anschlußschlauch (Konnektor) umschließt, wobei der mehrlumige Katheter aus den beiden Enden des Gehäuses nach außen vorsteht und das offene Ende eines Anschlußschlauches in dem Gehäuse bei einer seitlichen Wandlochung des mehrlumigen Katheters angeordnet ist.

Zum Anschluß einer einem Lumen des mehrlumigen Katheters zugeordneten Wandlochung an das innere offene Ende eines Anschlußschlauches werden bisher der Anschlußschlauch und der mehrlumige Katheter in zwei Gehäusehälften eines Katheteransatzes eingeklebt. Dies ist herstellungsmäßig problematisch und teuer. Hinzu kommt, daß sehr oft die Wandlochung des mehrlumigen Katheters und/oder das innere offene Ende des Anschlußschlauches verklebt sind, wodurch sich die Ausschußquote erhöht.

Wenn sich dieser Fehler erst bei der Anwendung zeigt, muß der gelegte Katheter herausgezogen und durch einen neuen Katheter ersetzt werden. Dies ist für den Anwender umständlich und für den Patienten schädlich. Ein nur teilweises Verkleben der Öffnungen ist äußerlich praktisch gar nicht feststellbar, hat aber durch Verringerung der Durchströmrate nachteiligen Einfluß auf die Infusion. Bei Kathetern mit mehr als zwei Lumina ist ihre Verbindung mit Anschlußschläuchen noch schwieriger, weil die verschiedene Flüssigkeiten oder Gase führenden Lumina zur Vermeidung einer Vermischung dieser Fluide keine gegenseitige Verbindung haben dürfen. Die Absperrung der einzelnen Lumina und zugehöriger Anschlußschlauchauslässe gegeneinander ist aber infolge von Undichtigkeiten an den Klebstellen bisher nicht ausreichend zuverlässig.

Der Erfindung liegt die Aufgabe zugrunde, einen Katheteransatz der erwähnten Art so auszubilden, daß bei einfacher und preiswerter Herstellbarkeit desselben alle Öffnungen des Katheters und des mindestens einen Anschlußschlauches zuverlässig offen bleiben und bei mehr als zwei Lumina des mehrlumigen Katheters diese in dem Gehäuse absolut zuverlässig und dicht voneinander getrennt sind.

Diese Aufgabe wird dadurch gelöst, daß das Gehäuse als einteiliges Kunststoffspritzgußteil um von Öffnungen freie Bereiche des mehrlumigen Katheters und des Anschlußschlauches herumgeformt und mit einer nach außen abgedichteten Kammer ausgestattet ist, in der das innere Ende des Anschlußschlauches und die Wandlochung des mehrlumigen Katheters angeordnet sind.

Diese Ausbildung des Katheteransatzes ermöglicht seine einfache und preiswerte Herstellung, wobei Verklebungen von Öffnungen des Katheters und/oder des Anschlußschlauches garantiert vermieden werden. Die Ausschußquote ist praktisch gleich Null. Alle Öffnungen sind zuverlässig frei und die Lumina haben die vorgegebene Durchströmrate, so daß ein mehrmaliges Verlegen von Kathetern entfällt und die Infusion durch korrekten Fluidstrom für den Patienten sicher ist. Die Herumformung des einteiligen Kunststoffspritzgußgehäuses um von Öffnungen freie Bereiche des Katheters und des Anschlußschlauches bewirkt eine selbsttätige hervorragende Abdichtung des Gehäuses an den Stellen der Schlauchdurchführungen.

Für Katheter mit mehr als zwei Lumina ist vorgesehen, daß in Richtung der Längsachse des Gehäuses mehrere Kammern hintereinander angeordnet und durch Zwischenwände voneinander getrennt sind und daß der mehrlumige Katheter und mindestens ein Anschlußschlauch durch die Zwischenwände hindurchragen, die um sie herumgespritzt sind. Da die Zwischenwände des Kunststoffspritzgußgehäuses dicht um den Katheter und die Anschlußschläuche herumgespritzt sind, ist eine absolute Trennung der Kammern und damit der Lumina des Katheters und der ihnen zugeordneten Anschlußschläuche voneinander gewährleistet. Eine Vermischung der zu infundierenden verschiedenen Flüssigkeiten oder Gase ist ausgeschlossen. Auch hierdurch wird die Sicherheit der Infusion erhöht.

Die hintereinanderliegenden Kammern sind identisch ausgebildet und der mehrlumige Katheter sowie der mindestens eine Anschlußschlauch liegen vorteilhafterweise in gleicher Ebene in dem flachgehaltenen Gehäuse.

In vorteilhafter Ausgestaltung der Erfindung ist vorgesehen, daß jede Kammer von einer Seitenwand des Gehäuses zur gegenüberliegenden Seitenwand durchgehend verläuft und in Fenstern in den Seitenwänden endet, die von dicht und fest eingesetzten Deckeln verschlossen sind. Die Deckel können eingeklebt oder eingeschweißt sein. Alternativ kann jede Kammer auf der einen Seite von einer geschlossenen Seitenwand des Gehäuses begrenzt sein und auf der gegenüberliegenden Seite in einem Fenster enden, das von einem dicht und fest eingesetzten Deckel verschlossen ist.

Gemäß dem Verfahren zur Herstellung des erfindungsgemäßen Katheteransatzes ist vorgesehen, daß man die Lumina des mehrlumigen Katheters und des mindestens einen Anschlußschlauches mit Metallkernen ausfüllt, daß man den mehrlumigen Katheter und den mindestens einen Anschlußschlauch so in die Rillen des Formnestes eines geöffneten Spritzgußwerkzeuges einlegt, daß eine Wandlochung des mehrlumigen Katheters und das innere offene Ende eines Anschlußschlauches etwa in der Mitte des Formnestes mindestens eines die Kammer bildenden Kernteiles des Spritzgußwerkzeuges liegen, und daß man das Spritzgußwerkzeug schließt und dann seine dem Körper des Gehäuses und ggf. Zwischenwände bildenden Hohlräume mit Kunststoff ausspritzt. Das ausgehärtete Gehäuse mit den eingelagerten Schläuchen wird dem geöffneten Spritzgußwerkzeug entnommen und es wird sodann jedes von einem Kernteil gebildete Fenster in einer Seitenwand des Gehäuses mit einem Deckel aus Kunststoff dicht und fest verschlossen. Der Katheteransatz mit mehrlumigem Katheter und mindestens einem Anschlußschlauch ist ohne Nachbearbeitung fertig und nach Sterilisierung einsatzbereit. Die Anwendung der Spritzgußtechnik erlaubt eine einfache und preiswerte Herstellung bei Gewährleistung absoluter Dichtigkeit der einzelnen Lumina gegeneinander und nach außen.

In der Zeichnung sind Ausführungsbeispiele der Erfindung schematisch dargestellt. Es zeigen:

Fig. 1 eine Seitenansicht eines Katheteransatzes mit einem zweilumigen Katheter,

Fig. 2 einen Einblick in den Katheteransatz von oben gesehen,

Fig. 3 eine Vorderansicht des Katheteransatzes in Richtung der Pfeile III-III in Fig. 1 gesehen,

Fig. 4 einen Querschnitt längs der Linie IV-IV in Fig. 1,

Fig. 5 das auseinandergefahrene Spritzgußwerkzeug zur Herstellung des Katheteransatzes nach Fig. 1 bis 4,

Fig. 6 einen Querschnitt längs der Linie VI-VI in Fig. 5,

Fig. 7 das zusammengefahrene Spritzgußwerkzeug nach Fig. 5,

Fig. 8 einen Querschnitt längs der Linie VIII-VIII in Fig. 7,

Fig. 9 einen Querschnitt durch das auseinandergefahrene Spritzgußwerkzeug zur Entformung,

Fig. 10 eine Seitenansicht eines Katheteransatzes mit einem dreilumigen Katheter,

Fig. 11 einen Einblick in den Katheteransatz nach Fig. 10 von oben gesehen und

Fig. 12 einen Querschnitt längs der Linie XII-XII in Fig. 10.

Der Katheteransatz 1 besteht aus einem Gehäuse 2, das einstückig als Kunststoffspritzgußteil ausgebildet ist. In dem Gehäuse 2 ist längsverlaufend ein zweilumiger Katheter 3 angeordnet, dessen großes, im Querschnitt halbkreisförmiges Lumen 3a von einem Ende des Katheters zum anderen durchgeht, während sein kleineres Lumen 3b mit kreisförmigem Querschnitt sich von der Spitze des Katheters 3 bis zu einer seitlichen Wandlochung 4 zwischen den Enden des Katheters 3 erstreckt. Der Katheter 3 ist gerade durch das Gehäuse 2 hindurchgeführt und ragt an beiden Enden weit über dieses vor. Während das große Lumen 3a außerhalb des Gehäuses 2 am rechten Ende des Katheters 3 an eine Versorgungsquelle, z.B. ein Übertragungsgerät für Infusionsflüssigkeit, angeschlossen ist, wird das kleine Lumen 3b innerhalb des Gehäuses 2 mit dem gewünschten Fluid, z.B. einer anderen Infusionsflüssigkeit oder dergleichen, versorgt. Zu diesem Zweck wird ein Anschlußschlauch 5 benutzt, dessen äußeres offenes Ende an eine Versorgungsquelle angeschlossen ist und dessen inneres offenes Ende 6 im Bereich der Wandlochung 4 des Katheters 3 endet. Die Wandlochung 4 und das offene Ende 6 befinden sich berührungsfrei in einer rechteckigen Kammer 7 des Gehäuses 2, in der ein ungehinderter Flüssigkeitsstrom aus dem offenen Ende 6 durch die Wandlochung 4 in das kleine Lumen 3b erfolgen kann.

Die Kammer 7 verläuft von einer geraden Seitenwand des Gehäuses 2 zur gegenüberliegenden geraden Seitenwand durchgehend und endet in beiden Seitenwänden in Fenstern 8 und 9, die von Deckeln 10 und 11 verschlossen sind. Die Deckel 10 und 11 greifen mit einem Absatz in Falzen der Seitenwände ein und sind zur Befestigung und Abdichtung eingeklebt oder eingeschweißt. Der Katheter 3 und der Anschlußschlauch 5 liegen in der Ebene zwischen den beiden Deckeln 10 und 11 übereinander und beide sind in der Kammer 7 so angeordnet, daß sie keine innere Gehäusewand berühren, so daß der Flüssigkeitsübergang von dem Anschlußschlauch 5 durch die Wandlochung 4 in das kleine Lumen 3b des Katheters 3 kontinuierlich erfolgt. Da Nischen, in denen sich Flüssigkeit sammeln könnte, fehlen, sind eine freie Flüssigkeitseinleitung in das kleine Lumen 3b und eine kontinuierliche Durchströmrate gewährleistet.

Ein sehr wesentliches Merkmal des erfindungsgemäßen Katheteransatzes 1 besteht darin, daß er sich preiswert herstellen läßt und absolut dicht ist. Dies wird dadurch erzielt, daß das Gehäuse 2 im Spritzgußverfahren hergestellt wird und daß der Spritzgußkörper unter Freihaltung der Kammer 7 und Offenhaltung des offenen Endes 6 und der Wandlochung 4 um den Katheter 3 und den Anschlußschlauch 5 unmittelbar herumgeformt wird. Ohne Klebstellen umschließt das einteilige Gehäuse 2 den Katheter 3 und den Anschlußschlauch 5 haftfest und dicht - nur der Raum für den Flüssigkeitsübertritt zwischen den beiden Öffnungen 4 und 6 bleibt frei.

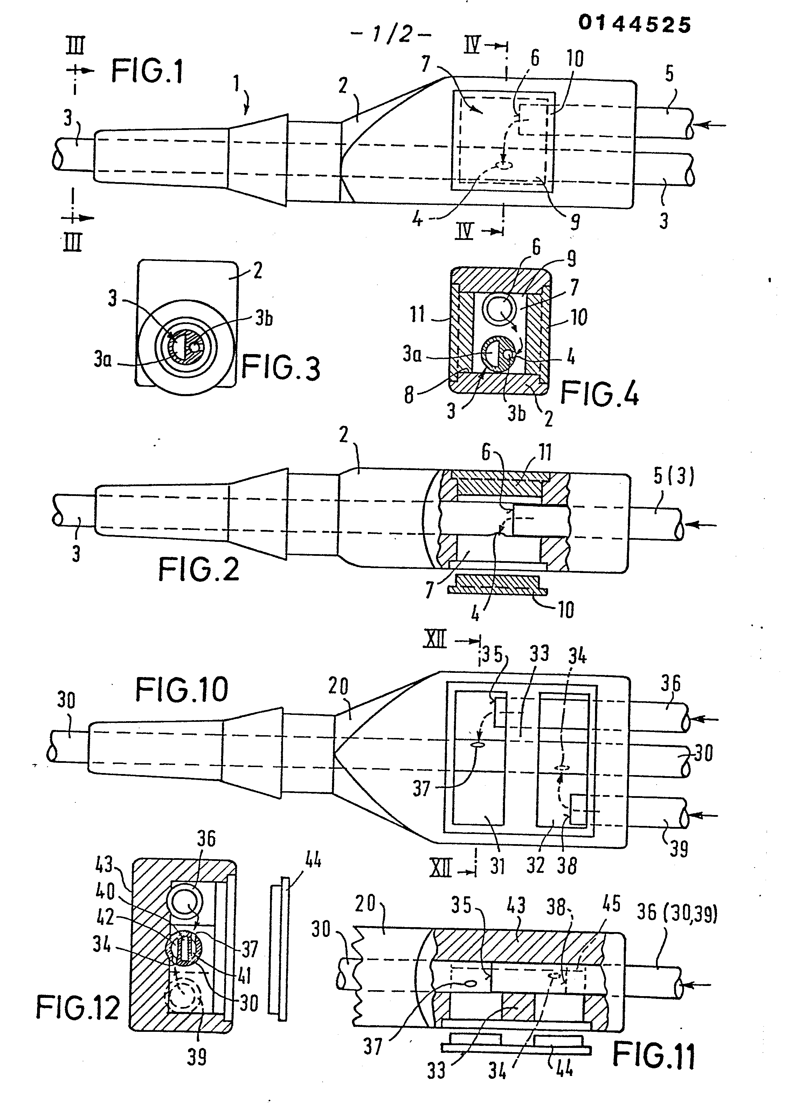

In den Fig. 4 bis 9 ist ein Schema der Spritzgußherstellung des Katheteransatzes 1 gemäß Fig. 1 bis 4 dargestellt.

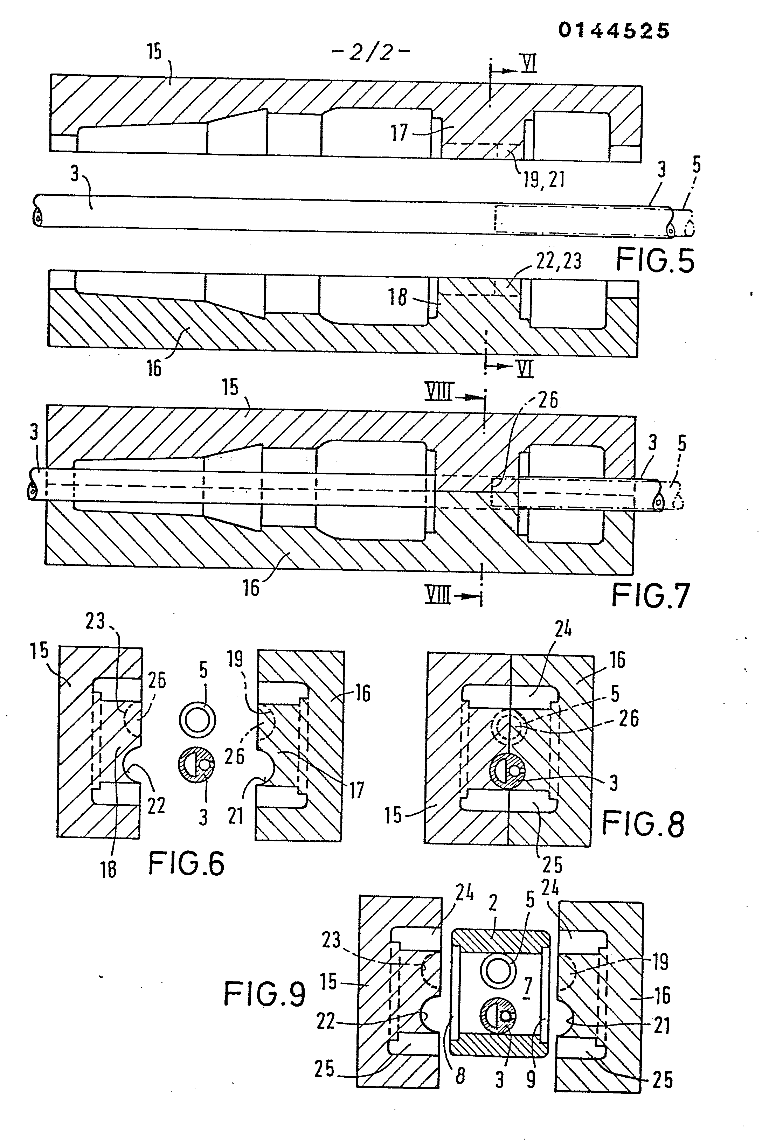

Es sind zwei Formenhälften 15, 16 vorgesehen, die die gewünschten Außenkonturen des Gehäuses 2 als Hohlform enthalten und die jeweils mit Kernteilen 17, 18 ausgestattet sind, die sich koaxial gegenüberliegen und die jeweils zwei parallele Formnester aufweisen (Figuren 5 und 6). Die Formnester sind Kanäle mit kreisförmigem Querschnitt, die in Längsrichtung des geschlossenen Spritzgußwerkzeuges verlaufen und aus im Querschnitt halbkreisförmigen Rillen 19,21; 22;23 zusammengesetzt sind, die den Anschlußschlauch 5 und den Katheter 3 passend aufnehmen (Fig. 8). Der Anschlußschlauch 5 liegt in den Formnestern der Rillen 19, 23 und der Katheter 3 befindet sich in den Formnestern der Rillen 21, 22. An den beiden quer zur Längsachse des Spritzgußwerkzeuges quer gerichteten Enden der Kernteile 17 und 18 sind flache Formhohlräume 24, 25 ausgebildet, die zueinander parallel sind und deren innere und äußere Begrenzung gerade verläuft. Sie dienen der Formung der geschlossenen Wände des Gehäuses 2 an der Kammer 7.

Zur Herstellung des Katheteransatzes 1 wird ein der Deutlichkeit halber nicht gezeichneter Stahlkern mit kreisförmigem Querschnitt in den Anschlußschlauch 5 eingeführt und es werden die beiden Lumina 3a und 3b des Katheters 3 mit Stahlkernen angepaßter Profilierung ausgefüllt. Das kleine Lumen 3b des Katheters 3 ist bereits mit der Wandlochung 4 versehen. Der Katheter 3 wird so in die Rillen 21, 22 des Formnestes eingelegt, daß sich die Wandlochung 4 ungefähr in der Mitte der Kernteile 17, 18 befindet. Sodann wird der Anschlußschlauch 5 bis zu einem Anschlag 26 am Ende der Rillen 19, 23 in diese eingelegt. Die beiden Formenhälften 15,16 werden geschlossen (Fig. 8) und in den Formenhohlraum wird Kunststoff eingespritzt. Dieser umgibt den Katheter 3 und den Anschlußschlauch 5 nur dort nicht, wo die Kernteile 17, 18 zur Bildung der Kammer 7 in dem Gehäuse 2 die Bereiche um die Öffnungen 4 und 6 fest umschließen und Kunststoffmaterial von diesen Bereichen fernhalten. Ein Zusetzen der Öffnungen 4 und 6 wird auf diese Weise verhindert und bei dem Katheteransatz 1, der den auseinandergefahrenen Formenhälften 15 und 16 entnommen wird (Fig. 9), befinden sich diese Öffnungen 4 und 6 in der Kammer 7 und ein freier Durchgang zwischen den Öffnungen 4 und 6 ist garantiert. Anschließend werden die beiden Fenster 8 und 9 des Gehäuses 2 mit den Deckeln 10 und 11 dicht verschlossen (Fig. 2).

Fig. 10, 11 und 12 zeigen einen dreilumigen Katheter 30, der mit einem Katheteransatz 20 ausgerüstet ist, dessen Herstellung ähnlich wie unter Bezug auf die Fig. 5 bis 9 erläutert, erfolgt, wobei allerdings die eine Seitenwand kein Fenster aufweist, sondern von vornherein geschlossen ist. Das Spritzgußwerkzeug zur Herstellung von zwei Kammern 31, 32 in einem Gehäuse 20 ist hinsichtlich der Kernteile entsprechend abgewandelt.

In dem Gehäuse 20 liegen die beiden Kammern 31, 32 auf der Längsachse des Gehäuses 20 hintereinander. Sie sind identisch und durch eine Zwischenwand 33, die ebenfalls gespritzt ist, abgedichtet voneinander getrennt. In der Kammer 31 befinden sich eine Wandlochung 34 des Katheters 30 und das offene Ende 35 eines Anschlußschlauches 36, während in der zweiten Kammer 32 eine zweite Wandlochung 37 des Katheters 30 und das offene Ende 38 eines zweiten Anschlußschlauches 39 angeordnet sind. Da die beiden Kammern 31 und 32 durch die Zwischenwand 33 dicht gegeneinander abgesperrt sind, besteht keine Verbindung zwischen den Wandlochungen 34 und 37 des Katheters 30, die verschiedenen Lumina des Katheters 30 zugeordnet sind, welche unterschiedliche Flüssigkeiten oder Gase führen, die nicht miteinander vermischt werden dürfen. Die drei Lumina 40, 41 und 42 des Katheters
30 sind in Fig. 12 erkennbar. Das mittlere Lumen 40
geht von einem Ende des Katheters 30 zum anderen durch,
während die beiden äußeren Lumina 41 und 42 von der
Spitze des Katheters 30 bis zu der Wandlochung 34 bzw.
der gegenüberliegenden Wandlochung 37 verlaufen. Der
Katheter 30 und die beiden ihn flankierenden Anschlußschläuche 36 und 39 liegen in einer gemeinsamen Ebene
des abgeflachten Gehäuses 20. Die eine Seitenwand 43
des Gehäuses 20 wird bei Herstellung des Katheteransatzes im Spritzgußverfahren geschlossen, d.h ohne
Fenster ausgebildet, während in der gegenüberliegenden
Seitenwand zwei Fenster nebeneinanderliegen, die jeweils einer Kammer 31 bzw. 32 zugeordnet sind und die
mittels eines eingeklebten oder eingeschweißter Deckels
44 verschlossen sind, welcher zwei in die Fensteröffnungen passende Einsätze trägt (Fig. 11, 12).

Die Seitenwand 43 besitzt auf ihrer Innenfläche flache
Rillenvertiefungen 45, in die der Katheter 30 und die
Anschlußschläuche 36, 39 eingebettet sind. Die Innenfläche des Deckels 44 ist unprofiliert eben und befindet sich im Abstand zu dem Katheter 30 und den Anschlußschläuchen 36, 39, damit ein freier Fluidstrom
zwischen diesen Teilen möglich ist.

ANSPRÜCHE

1. Katheteransatz, bestehend aus einem Gehäuse, das einen mehrlumigen Katheter und mindestens einen zu diesem parallelen Anschlußschlauch (Konnektor) umschließt, wobei der mehrlumige Katheter aus den beiden Enden des Gehäuses nach außen vorsteht und das eine offene Ende eines Anschlußschlauches in dem Gehäuse bei einer seitlichen Wandlochung des mehrlumigen Katheters angeordnet ist,
dadurch gekennzeichnet,
daß das Gehäuse (2;20) als einteiliges Kunststoffspritzgußteil um von Öffnungen (4,6;34,35;37,38) freie Bereiche des mehrlumigen Katheters (3;30) und des Anschlußschlauches (5;36,39) herumgeformt und mit einer nach außen abgedichteten Kammer (7;31,32) ausgestattet ist, in der das innere Ende des Anschlußschlauches (5;36,39) und die Wandlochung (4;34,37) des mehrlumigen Katheters (3;30) angeordnet sind.

2. Katheteransatz nach Anspruch 1,
dadurch gekennzeichnet,
daß in Richtung der Längsachse des Gehäuses (20) mehrere Kammern (31,32) hintereinander angeordnet und durch Zwischenwände (33) voneinander getrennt sind und daß der mehrlumige Katheter (30) und mindestens ein Anschlußschlauch (36,39) durch die Zwischenwände (33) hindurchragen, die um sie herumgespritzt sind.

3. Katheteransatz nach Anspruch 2 ,
dadurch gekennzeichnet,
daß die hintereinanderliegenden Kammern (31,32) identisch ausgebildet sind.

4. Katheteransatz nach einem der Ansprüche 1 bis 3,
d a d u r c h   g e k e n n z e i c h n e t ,
daß jede Kammer (7) von einer Seitenwand des Gehäuses
(2) zur gegenüberliegenden Seitenwand durchgehend verläuft und in Fenstern (8,9) in den Seitenwänden endet,
die von dicht und fest eingesetzten Deckeln (10,11)
verschlossen sind.

5. Katheteransatz nach Anspruch 4,
d a d u r c h   g e k e n n z e i c h n e t ,
daß die Deckel (10,11) eingeklebt oder eingeschweißt
sind.

6. Katheteransatz nach einem der Ansprüche 1 bis 3,
d a d u r c h   g e k e n n z e i c h n e t ,   daß
jede Kammer (31,32) auf der einen Seite von einer geschlossenen Seitenwand (43) des Gehäuses (20) begrenzt
ist und auf der gegenüberliegenden Seite in einem Fenster endet, das von einem dicht und fest eingesetzten
Deckel (44) verschlossen ist.

7. Katheteransatz nach einem der Ansprüche 1 - 6,
d a d u r c h   g e k e n n z e i c h n e t ,
daß der mehrlumige Katheter (3;30) und der mindestens
eine Anschlußschlauch (5;36,39) in gleicher Ebene in
dem Gehäuse (2;20) nebeneinanderliegen.

8. Verfahren zur Herstellung eines Katheteransatzes
nach den Ansprüchen 1 bis 7,
d a d u r c h   g e k e n n z e i c h n e t ,
daß man die Lumina des mehrlumigen Katheters und des
mindestens einen Anschlußschlauches mit Metallkernen
ausfüllt, daß man den mehrlumigen Katheter und den mindestens einen Anschlußschlauch so in Rillen des Formnestes eines geöffneten Spritzgußwerkzeuges einlegt, -

daß eine Wandlochung des mehrlumigen Katheters und das innere offene Ende eines Anschlußschlauches etwa in der Mitte des Formnestes mindestens eines die Kammer bildenden Kernteiles des Spritzgußwerkzeuges liegen und daß man das Spritzgußwerkzeug schließt und dann seine den Körper des Gehäuses und ggf. Zwischenwände bildenden Hohlräume mit Kunststoff ausspritzt.

9. Verfahren nach Anspruch 8,
d a d u r c h   g e k e n n z e i c h n e t,
daß man das Gehäuse aus dem geöffneten Spritzgußwerkzeug entnimmt und jedes von einem Kernteil gebildete Fenster in einer Seitenwand des Gehäuses mit einem Deckel dicht und fest verschließt.

0144525

-1/2-

FIG.1
FIG.3
FIG.4
FIG.2
FIG.10
FIG.12
FIG.11

0144525

-2/2-

FIG.5

FIG.7

FIG.6

FIG.8

FIG.9